# EUROPÄISCHE PATENTANMELDUNG

(11) **EP 3 725 334 A1**
(43) Veröffentlichungstag der Anmeldung: **21.10.2020**
(21) Anmeldenummer: 19169430.6
(22) Anmeldetag: 16.04.2019
(51) Int. Cl.: A61L 2/08, A61L 2/10, A61L 2/18, A61L 2/20, A61L 2/22, C02F 1/32

(54) **VORRICHTUNG UND VERFAHREN ZUR REDUZIERUNG DER ANZAHL VON KEIMEN**

(71) Anmelder: HUBL GmbH Edelstahltechnik, 71665 Vaihingen/Enz (DE)
(72) Erfinder: Kiefer, Rainer, 71254 Ditzingen (DE)
(74) Vertreter: Jeck, Anton

(57) **Zusammenfassung**

Die vorliegende Erfindung betrifft ein Verfahren zur Reduzierung der Anzahl von Keimen in einem Medium, insbesondere Ölen, flüssig oder gasförmig bei welchem ein Reinigungsfluid dem Medium zum Bilden eines Gemisches beigemengt wird, das Gemisch mit einer elektromagnetischen Strahlung mit mindestens einer definierten Wellenlänge gleichmäßig bestrahlt wird und das Reinigungsfluid im Zuge der Bestrahlung des Gemisches die Keime reduziert. Weiterhin betrifft die vorliegende Erfindung eine Vorrichtung zur Reduzierung von Keimen in einem Medium, insbesondere Ölen, flüssig oder gasförmig mit mindestens einem Durchflussbehälter, durch den das Medium mit beigemischtem Reinigungsfluid transportierbar ist, und mindestens einer Strahlungsquelle, die ausgebildet ist, das transportierte Gemisch mit einer Strahlung definierter Wellenlänge gleichmäßig zu bestrahlen.

## Beschreibung

Die Erfindung betrifft ein Verfahren zur Reduzierung der Anzahl von Keimen in oder auf einem Medium gemäß Anspruch 1 sowie eine Vorrichtung zur Reduzierung von Keimen in oder auf einem Medium gemäß Anspruch 9.

Dem Fachmann sind eine Vorrichtung sowie ein Verfahren bekannt, bei welchen ein zu desinfizierendes Objekt durch eine Vorreinigung geschleust wird, wobei in der Vorreinigungsvorrichtung das zu entkeimende Objekt gereinigt und anschließend mit einem Reinigungsfluid besprüht wird. Durch Bestrahlung wird eine Desinfizierung der Oberfläche erreicht.

Der vorliegenden Erfindung liegt daher die **Aufgabe** zugrunde, eine Vorrichtung sowie ein Verfahren zur effizienten Reduzierung von Keimen in fluiden Medien und auf Oberflächen zu entwickeln.

Die Aufgabe wird zum einen durch ein Verfahren gemäß dem Verfahrensanspruch 1 sowie durch eine Vorrichtung gemäß dem Vorrichtungsanspruch 9 gelöst.

Vorteilhafte Ausführungsformen der vorliegenden Erfindung sind in den Ansprüchen angegeben.

Das erfindungsgemäße Verfahren ist dadurch gekennzeichnet, dass ein Reinigungsfluid dem Medium zum Bilden eines Gemisches beigemengt wird und/oder auf diese aufgetragen wird, das Gemisch beziehungsweise die benetzte Oberfläche mit einer elektromagnetischen Strahlung mit mindestens einer definierten Wellenlänge gleichmäßig bestrahlt wird und das Reinigungsfluid im Zuge der Bestrahlung des Gemisches die Keime reduziert.

Weiterhin ist die erfindungsgemäße Vorrichtung dadurch gekennzeichnet, dass diese mindestens einen Durchflussbehälter, durch den das Medium mit beigemischtem Reinigungsfluid transportierbar ist, und mindestens eine Strahlungsquelle aufweist, die ausgebildet ist, das transportierte Gemisch mit einer Strahlung definierter Wellenlänge gleichmäßig zu bestrahlen.

Das Reinigungsfluid kann grundsätzlich ein Gas, ein Aerosol ein Rauch, ein Pulver, eine Lösung sein oder in flüssiger Form vorliegen. Das Reinigungsfluid kann auch als Reinigungsmittel bezeichnet werden, muss den oder die maßgeblichen Wirkstoffe jedoch nicht in Reinform, sondern beispielsweise auch verdünnt oder als Lösung enthalten.

Erfindungsgemäß kann mit "gleichmäßig" nach Anspruch 1 insbesondere eine über die Oberfläche gleichmäßig verteilte Bestrahlung oder bei einem Fluid die Gleichmäßige Bestrahlung aller Anteile, oder zumindest der meisten Anteile, des Gemischs verstanden werden. Zumindest die Bestandteile des Reinigungsfluids, die bestrahlt werden, können eine Reinigungswirkung entfalten.

Ein Grundgedanke der vorliegenden Erfindung besteht darin, ein mit Keimen belastetes Medium, insbesondere ein Fluid, vorzugsweise ein Öl, einem wässrigen Medium, oder ein Gas, mit einer Substanz, vorzugsweise einem Reinigungsfluid, zu versetzen, das gleichmäßig in dem Medium vorsehbar, vorzugsweise lösbar oder dispergier oder suspendierbar ist, um dieses Gemisch einer Strahlungsquelle auszusetzen, insbesondere einer Strahlungsquelle für elektromagnetische Strahlung, vorzugsweise ein Leuchtmittel für sichtbares Licht. Hierdurch wird eine Verringerung der Anzahl der Keime in dem Medium erreicht. Dabei kommt es vorzugsweise zu einer Wechselwirkung zwischen der Strahlung und der Substanz, wodurch diese aktiviert wird und die aktivierte Substanz zur Entkeimung des Mediums beiträgt. Somit kann eine doppelte Wirkung der Strahlung erreicht werden, nämlich eine direkte Einwirkung der Strahlung auf die Keime und eine daraus resultierende Reduzierung der Anzahl der Keime sowie eine Aktivierung der Substanz in dem Medium und eine anschließende Reduzierung der Anzahl der Keime mittels die aktivierte Substanz.

Erfindungsgemäß ist unter einer reduzierten Anzahl von Keimen die Anzahl von pathogenen Keimen, wie beispielsweise Viren, Bakterien oder Pilze, in ihrer aktiven Form zu verstehen. Zwar können die Keime nach der erfindungsgemäßen Reduzierung ihrer Anzahl immer noch in dem Medium vorliegen. Ihre Wirkung auf biologisches Material, insbesondere auf Menschen oder Tiere, kann jedoch dennoch eingeschränkt oder aufgehoben sein. Somit wird erfindungsgemäß die Anzahl der Keime in dem Medium betrachtet, welche "lebend" bzw. biologisch aktiv vorliegt. Es handelt sich demnach um einen "Abtötungsprozess", bei welchem die Zahl der "toten" beziehungsweise inaktiven Keime bei der erfindungsgemäß betrachteten reduzierten Anzahl keine Berücksichtigung findet.

Grundsätzlich kann die Substanz, welche nachfolgend als Reinigungsfluid bezeichnet wird, sowohl vor als auch nach der Verkeimung dem Medium beigemengt werden. Demnach entsteht die entkeimende Wirkung des Reinigungsfluids besonders durch seine strahlungsbedingte Aktivierung. Nach einer bevorzugten Ausführungsform kann das Reinigungsfluid unmittelbar vor (oder während) der Bestrahlung dem Medium beigemengt werden.

Die Wechselwirkung zwischen der Strahlung und dem Reinigungsfluid, welche zu einer Aktivierung des Reinigungsfluids führt, kann insbesondere eine selektive Wechselwirkung sein. Vorzugsweise sind einzelne oder eine Kombination von einzelnen Wellenlängen des elektromagnetischen Strahlungsspektrums geeignet, die Aktivierung des Reinigungsfluids zu bewirken. Hierbei kann es sich um die Strahlung mit einer definierten Wellenlänge oder um eine Strahlung mit einer Kombination von Wellenlängen handeln. Grundsätzlich kann es mehr als eine Wellenlänge geben, welche jeweils einzelnen oder in Kombination mit weiteren Wellenlängen geeignet ist, das Reinigungsfluid zu aktivieren. Hierbei kann erfindungsgemäß eine definierte Wellenlänge sowohl eine diskrete Wellenlänge X nm ± 0 nm als auch ein Wellenlängenbereich sein (X nm ± Y nm; wobei Y zwischen 0,1 und 50 nm ist), in welchem eine Aktivierung des Reinigungsfluids möglich ist. X kann insbesondere 405 nm sein. Vorzugsweise handelt es sich um Licht, also eine elektromagnetische Strahlung im sichtbaren Bereich, insbesondere im Bereich von 380 bis 780 nm.

Das Reinigungsfluid kann beispielsweise 10H-Benzo[g]pteridin-2,4-dion-Derivate als aktiven Bestanteil aufweisen.

Nach einer bevorzugten Ausführungsform der vorliegenden Erfindung ist vorgesehen, dass das geförderte Gemisch als Film im Bereich einer Strahlungsquelle bereitgestellt wird. Ein Film kann insbesondre eine dünne Fluidschicht sein, welche an der Strahlungsquelle oder den Strahlungsquellen der elektromagnetischen Strahlung vorbeigeführt werden können. Grundsätzlich kann der Film auch Teil einer festen Schicht sein, welche auf einer Oberfläche aufgetragen sein kann. Hierbei kann es sich beispielsweise um eine mit dem Reinigungsfluid versetzte Wandfarbe handeln. Durch die Begrenzung der Schichtdicke des bestrahlten Mediums, welches vorzugsweise als eine Flüssigkeit vorliegt, kann nicht nur flächig sondern auch in der Tiefe des Films eine ausreichende Bestrahlung des Gemisches gewährleistet werden. Grundsätzlich nimmt die Strahlungsintensität aufgrund des Absorptionsverhaltens eines Mediums mit steigender Eindringtiefe in das Medium ab. Sowohl aus Gründen des Energieeinsatzes als auch der Geschwindigkeit, mit welcher das Reinigungsfluid aktivierbar ist, ist es daher sinnvoll, die Schichtdicke des bestrahlten Gemisches möglichst klein zu halten.

Erfindungsgemäß kann ein Film eine beliebige Form aufweisen. Wesentliches Element eines erfindungsgemäßen Films kann jedoch seine Schichtdicke sein. Bevorzugt ist es, dass der Film eine Dicke von 0,1 mm bis 30 cm, vorzugsweise von 5 mm bis 5 cm, aufweist.

Besonders bevorzugt ist eine Weiterbildung der Erfindung, wonach das Reinigungsfluid für eine Reduzierung der Keime durch elektromagnetische Strahlung aktiviert wird. Zwar kann die Strahlung grundsätzlich geeignet sein, eine Reduzierung der Anzahl der Keime hervorzurufen, das Reinigungsfluid hingegen entfaltet seine entkeimende bzw. reduzierende Wirkung jedoch vorzugsweise erst nach Aktivierung durch die auf das Gemisch einwirkende Strahlung. Grundsätzlich können unterschiedliche Strahlungsquellen vorgesehen sein, so dass zum einen eine Strahlung bereitgestellt sein kann, das Reinigungsfluid zu aktivieren, und zum anderen eine weitere Strahlungsquelle vorgesehen sein kann, um die Anzahl der Keime in dem Medium direkt zu reduzieren.

Besonders zweckmäßig ist es nach einer Weiterbildung der Erfindung, dass die Anzahl der Keime durch einen Kontakt mit dem Reinigungsfluid reduziert wird. Vorzugsweise ist demnach vorgesehen, dass durch eine Interaktion zwischen dem Reinigungsfluid und den Keimen ein Abtöten der Keime bewirkt wird.

Zur Regelung der Förderung des Gemisches zu der elektromagnetischen Strahlung kann nach einer vorteilhaften Weiterbildung der Erfindung vorgesehen sein, dass das Medium durch eine Sogwirkung oder einen erhöhten Druck einer Pumpe gefördert wird. Insbesondere durch die Sogwirkung kann einem Überdruck in einer Vorrichtung, in welcher das Gemisch der elektromagnetischen Ausstrahlung ausgesetzt wird, vorgebeugt werden.

Besonders zweckmäßig ist es nach einer Weiterbildung der Erfindung, dass eine Intensität der elektromagnetischen Strahlung angepasst wird, um in dem Medium eine Mindestintensität zu erreichen. Zusätzlich zu der mindestens einen Wellenlänge kann die Aktivierung des Reinigungsfluids auch von der Intensität der vorgesehenen Strahlung beziehungsweise einer Bestrahlungsdauer abhängen. Werden diese Parameter nicht berücksichtigt, kann das Fluid nach einer Bestrahlung inaktiv vorliegen. Die Intensität der Strahlung beziehungsweise die Bestrahlungsdauer kann insbesondere von der Schichtdicke des Films abhängen. Je größer die Schichtdicke des Films im Bereich der Strahlung vorgesehen ist, desto höher ist die erforderliche Intensität der einwirkenden Strahlung, um eine Aktivierung des Reinigungsfluids zu gewährleisten. Grundsätzlich sind die Absorptionseigenschaften des Gemisches zu berücksichtigen, die je nach Medium variieren können.

Eine besonders bevorzugte Weiterbildung der vorliegenden Erfindung sieht vor, dass das Medium mit einer Fördergeschwindigkeit zu der Quelle der elektromagnetischen Strahlung bereitgestellt wird, welche an die Intensität der elektromagnetischen Strahlung angepasst wird. Grundsätzlich kann die Aktivierung des Reinigungsfluids zusätzlich oder alternativ zu der Wellenlänge auch von einer Bestrahlungsdauer abhängen. Diese kann insbesondere mit der Strahlungsintensität korrelieren. Je höher die Strahlungsintensität ist, desto höher kann die Fördergeschwindigkeit des Gemisches vorgesehen werden, wobei dennoch zumindest ein wesentlicher Teil des Reinigungsfluids aktiviert wird. Ist die Intensität der Strahlung geringer und/oder die Schichtdicke des Mediums größer, ist eine verminderte Fördergeschwindigkeit erforderlich, um eine ausreichende Bestrahlungszeit für eine Aktivierung des Reinigungsfluids zu gewährleisten.

Ein weiterer Grundgedanke der vorliegenden Erfindung zur Reduzierung von Keimen in einem Medium besteht darin, eine Vorrichtung mit einem Durchflussbehälter und einer Strahlungsquelle vorzusehen. Mittels des Durchflussbehälters kann das Medium der Strahlungsquelle(n) zugeführt werden, beziehungsweise in einem Bereich gefördert werden, welcher von der Strahlungsquelle bestrahlt wird. Das Medium kann hierbei mit dem Reinigungsfluid zu einem beliebigen Zeitpunkt durchmischbar sein. Eine entsprechende Einrichtung zum Zuführen des Reinigungsfluids zu dem Medium kann beispielsweise im Bereich des Durchflussbehälters, vorzugsweise in Strömungsrichtung vor oder in unmittelbarer Nähe der Strahlungsquelle, angeordnet sein. Besonders bevorzugt ist das Medium mit dem Reinigungsfluid als Gemisch dem Durchflussbehälter zuführbar. Bevorzugt ist der Durchflussbehälter als Strömungseinrichtung ausgebildet, durch welchen das Medium, insbesondere mit beigemischtem Reinigungsfluid, strömbar ist. Die Strahlungsquelle kann vorzugsweise als statische Einrichtung vorgesehen sein, welcher vorzugsweise kontinuierlich das Medium mit dem Reinigungsfluid mittels des Durchflussbehälters zuführbar ist. Die Strahlungsquelle kann ausgebildet sein, um eine elektromagnetische Strahlung zumindest einer spezifischen Wellenlänge zu imitieren. Dabei ist die zumindest eine spezifische Wellenlänge auf das Reinigungsfluid abstimmbar. Vorzugsweise entspricht die emittierte Wellenlänge der Wellenlänge, welche zur Aktivierung des Reinigungsfluids erforderlich ist. Es können auch ein Wellenlängenbereich, mehrere Wellenlängenbereiche oder eine Anzahl von zumindest zwei Wellenlängen ausgebildet sein, um eine Aktivierung des Reinigungsfluids zu bewirken.

Nach einer bevorzugten Weiterbildung der erfindungsgemäßen Vorrichtung ist vorgesehen, dass der Durchflussbehälter dafür ausgebildet ist, das keimbelastete Medium im Bereich der Strahlungsquelle zu fördern. Dabei ist ein formgebendes Element vorgesehen, welches das Medium, soweit es sich um ein fluides medium handelt, im Bereich der Strahlungsquelle, also in einem von die Strahlungsquelle bestrahlten Bereich, als Film bereitstellt. Grundsätzlich kann der Durchflussbehälter als formgebendes Element ausgebildet sein. Das formgebende Element kann insbesondere ausgebildet sein, um das transportierte Medium mit einer Schichtdicke bereitzustellen, welche ausreichend dünn ist, eine Aktivierung des Reinigungsfluids zu bewirken, vorzugsweise im gesamten der Strahlungsquelle zugestellten Medium.

Gemäß den voranstehenden Ausführungen ist bevorzugt, eine Mindestintensität der Strahlung an der Strahlungsquelle beziehungsweise in dem Medium vorzusehen, welche ausreichend ist, eine Aktivierung des zugestellten Reinigungsfluids zu bewirken.

Die Geometrie des formgebenden Elements kann hierbei an die Durchflussmenge durch den mindestens einen Durchflussbehälter, die Strömungsgeschwindigkeit, die Anzahl an Strahlungsquellen und/oder eine Konzentration an Reinigungsfluid in dem Medium anpassbar sein.

Erfindungsgemäß kann als Film eine Schichtdicke des durch den Durchflussbehälter strömenden Mediums bestimmt sein, welche zumindest im Bereich des formgebenden Elements eine vorzugsweise gleichmäßige Aktivierung des Reinigungsfluids in dem Medium ermöglicht. Hierbei ist die Form des formgebenden Elements bzw. des Films nicht auf eine Ebene der Ausgestaltung beschränkt. Der Film beziehungsweise das formgebende Element kann erfindungsgemäß insbesondere in Form eines flachen Quaders, eines Zylinders (Rohr), insbesondere eines Hohlzylinders, oder einer Bündelung eines oder mehrerer geometrischer Ausgestaltungen von Teilfilmen bzw. teilformgebenden Elementen ausgebildet sein. Hinsichtlich der Teilfilme bzw. teilformgebenden Elemente kann beispielsweise eine Anzahl von vorzugsweise dünnen Röhren vorgesehen sein, welche bevorzugt gebündelt angeordnet sind. Dabei kann die mindestens eine Strahlungsquelle mit einem bevorzugt gleichmäßigen Abstand zu den einzelnen Röhren vorgesehen sein.

Nach einer bevorzugten Weiterbildung der vorliegenden Erfindung ist vorgesehen, dass das formgebende Element als Doppelschlitz ausgebildet ist, durch welchen das Medium förderbar ist. Mittels eines Doppelschlitzes kann das Medium insbesondere als dünner Film bereitgestellt werden, wobei die Strahlungsquelle bevorzugt unmittelbar im Bereich des formgebenden Elements beziehungsweise des Films vorgesehen sein kann. Der Doppelschlitz kann mit zwei nahe beieinanderliegenden Platten gebildet sein, zwischen welchen das fluide Medium bzw. das Gemisch strömen kann. Dabei kann der Abstand der Platten zu einander die Schichtdicke bzw. Filmdicke des Mediums bzw. Gemischs vorgeben.

Grundsätzlich kann erfindungsgemäß auch dann von einem Film die Rede sein, wenn ein Rohr bereit gestellt ist, durch welches das Gemisch oder Medium im Bereich der Strahlungsquelle strömt. Hierbei kann es sich insbesondere um ein dünnes Rohr von wenigen cm oder mm handeln. Vorzugsweise weist ein solches Rohr einen Durchmesser von weniger als 30 cm, 20 cm, 10 cm, 5 cm, besonders bevorzugt von weniger als 1 cm auf. "Im Bereich der Strahlungsquelle" kann insbesondere den Teil der Vorrichtung, des Doppelschlitzes oder der Rohre bezeichnen, welcher von er Strahlungsquelle bestrahlt wird wodurch die Zahl der lebenden Keime reduziert wird.

Die Strahlungsquelle kann insbesondere dazu ausgebildet sein, das Medium nach der Formgebung durch das formgebende Element zu bestrahlen. Die Strahlungsquelle kann hierbei insbesondere an die Form des Films angepasst werden und beispielsweise als längliche Strahlungsquelle ausgebildet sein, welche sich vorzugsweise quer zu einer Strömungsrichtung erstreckt.

Erfindungsgemäß können ebenfalls mehrere Doppelschlitze übereinander oder nebeneinander vorgesehen sein, wobei die mindestens eine Strahlungsquelle zwischen den einzelnen Doppelschlitzen beziehungsweise oberhalb und unterhalb der einzelnen Doppelschlitze vorsehbar sein kann.

Besonders bevorzugt ist es nach einer Weiterbildung der Erfindung, dass das formgebende Element als koaxiales Doppelrohr ausgebildet ist. Dabei ist die Strahlungsquelle im inneren Rohr vorgesehen, und das Medium ist durch das äußere Rohr förderbar. Eine oder mehrere solcher Doppelrohre können vorgesehen sein, den Durchflussbehälter der erfindungsgemäßen Vorrichtung bereitzustellen. Hierdurch kann auf engstem Raum eine Vielzahl von dünnen Oberflächenfilmen bereitgestellt sein. Durch die koaxiale Anordnung eines Innenrohrs und eines Außenrohrs kann selbst eine punktuell vorgesehene Strahlungsquelle eine große Oberfläche des strömenden Fluids bestrahlen.

Besonders zweckmäßig ist es nach einer Weiterbildung der Erfindung, dass das formgebende Element als eine Anordnung von mehreren Rohren ausgebildet ist, durch welche das Medium förderbar ist. Diese Rohre können insbesondere parallel zueinander oder kreisförmig angeordnet sein, wobei die mindestens eine Strahlungsquelle zwischen den einzelnen Rohren vorsehbar ist. Zusätzlich oder alternativ hierzu kann eine Strahlungsquelle mehrere Rohre zumindest an einer Stelle radial umgeben. Somit kann eine Bestrahlung aus mehreren Richtungen auf das Medium in den Rohren bewirkt werden.

Für eine besonders effiziente Bestrahlung des Mediums in dem Durchflussbehälter bzw. dem formgebenden Element ist nach einer Weiterbildung vorgesehen, dass mehrere Rohre, zumindest das innere Rohr des Doppelrohrs bzw. der Doppelschlitz, strahlungsdurchlässig für die Strahlung der mindestens einen Strahlungsquelle sind. Bevorzugt handelt es sich um ein Material, welches transparent ist und/oder einen geringen Absorptionskoeffizienten für die Strahlung der Strahlungsquelle aufweist, welche letztlich ausgebildet ist, um das Reinigungsfluid zu aktivieren. Grundsätzlich kann das Material ein Glas, ein Polymer bzw. ein Kunststoff, ein Harz, ein Naturstoff oder eine Kombination der genannten Materialien sein. Erfindungsgemäß ist grundsätzlich jedes Material geeignet, welches formstabil genug ist, das Fördern eines Mediums unter Unterdruck oder Überdruck durch den Durchflussbehälter bzw. das formgebende Element zu ermöglichen, ohne dass das Material Schäden dabei erleidet. Die mindestens eine Strahlungsquelle kann zumindest in einem Bereich des Durchflussbehälters vorgesehen sein.

Die Erfindung wird nachfolgend anhand der beigefügten schematischen Zeichnung näher erläutert. Es zeigen:
- Fig. 1: eine schematische Darstellung der erfindungsgemäßen Vorrichtung,
- Fig. 2: eine schematische Querschnittsansicht eines erfindungsgemäßen formgebenden Elements als Doppelschlitz,
- Fig. 3: eine schematische Querschnittsansicht eines erfindungsgemäßen formgebenden Elements als koaxiales Doppelrohr,
- Fig. 4: eine schematische Querschnittsansicht eines erfindungsgemäßen formgebenden Elements als Leitungsbündel von Rohren und
- Fig. 5: eine schematische Querschnittsansicht eines erfindungsgemäßen formgebenden Elements als Leitungsbündel von koaxialen Doppelrohren.

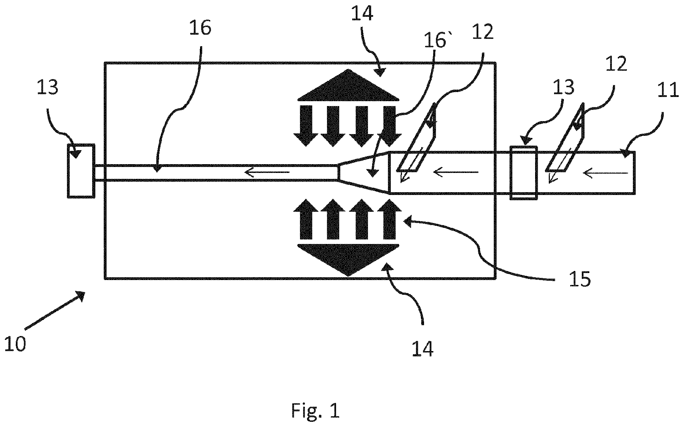

Fig. 1 zeigt eine erfindungsgemäße Vorrichtung 10 zur Reduzierung von Keimen in einem Medium. Die Anlage ist vorzugsweise mit einem Durchflussbehälter 11 gebildet, durch welchen das Medium leitbar ist. Das Medium kann bereits mit Reinigungsfluid vermischt sein, wenn es dem Durchflussbehälter 11 zugeführt wird. Alternativ kann das Reinigungsfluid über entsprechende Zufuhrkanäle 12 beziehungsweise Zufuhreinrichtungen 12 dem Medium in dem Durchflussbehälter 11 beigemischt werden. Mittels mindestens einer Pumpeinrichtung 13, welche stromaufwärts und/oder stromabwärts an dem Durchflussbehälter 11 angeordnet sein kann, kann das Medium durch die Vorrichtung bewegt werden. Hierbei liegt an dem Medium ein Unterdruck bzw. ein Überdruck an, je nachdem, wo im System der Vorrichtung 10 die Pumpeinrichtung 13 vorgesehen ist.

Zumindest in einem Bereich kann das Leitungssystem, durch welches das Medium geleitet wird, insbesondere der Durchflussbehälter 11, als formgebendes Element 16 ausgebildet sein. Zumindest jedoch ist das formgebende Element 16 in einem Bereich vorgesehen, in welchem mindestens eine Strahlungsquelle 14 angeordnet ist, welche ausgebildet ist, die Strahlung 15, insbesondere die elektromagnetische Strahlung, dem Medium mit Reinigungsfluid dem Durchflussbehälter 11 bzw. dem formgebenden Element 16 zuzuführen.

Grundsätzlich kann der Durchflussbehälter 11 als formgebendes Element 16 durchgehend in der Vorrichtung 10 vorgesehen sein. Ist der Durchflussbehälter 11 jedoch nur bereichsweise als formgebendes Element 16 ausgebildet, kann zumindest in einem Bereich ein Übergangselement 16' vorgesehen sein, welches das zu fördernde Medium als Film in dem formgebenden Element 16 bereitstellt. Das Zuführen des Reinigungsfluids kann an einem beliebigen Punkt stromaufwärts von der Strahlungsquelle 14 an dem Durchflussbehälter 11 bzw. an dem formgebenden Element 16 e-folgen sein. Vorzugsweise kann eine Durchmischung des Mediums mit dem Fluid und entsprechend eine Zufuhreinrichtung 12 direkt im Bereich des formgebenden Elements 16 angeordnet sein, welches durch die Strahlung 15 der Strahlungsquelle 14 bestrahlt wird.

Die Strömung des Mediums durch die Vorrichtung 10 ist durch die horizontal dargestellten Pfeile in Fig. 1 angedeutet. Das durch die Zufuhreinrichtungen 12 einströmende Reinigungsfluid ist durch die schräg dargestellten Pfeile angedeutet. Grundsätzlich ist eine Vielzahl von Anordnungen der mindestens einen Strahlungsquelle und dem formgebenden Element 16 zueinander denkbar. Nachfolgend sollen beispielhaft einige der geometrischen Ausgestaltungen des Durchflussbehälters als formgebendes Element erläutert werden.

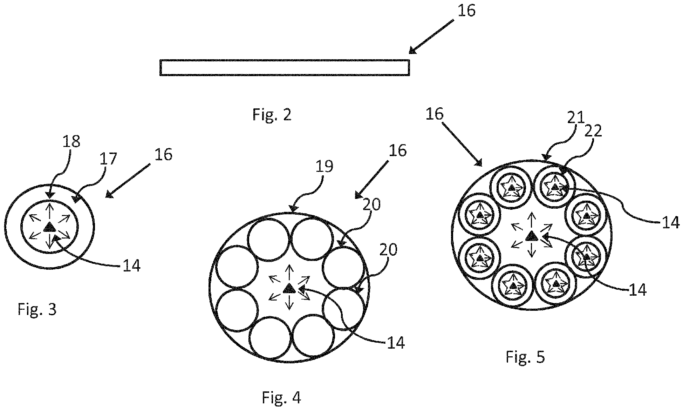

Fig. 2 zeigt das formgebende Element 16 als Doppelschlitz-Anordnung, wobei das Medium durch den länglichen Schlitz gefördert werden kann. Hierdurch kann das Medium mit einer besonders kleinen Filmdicke durch die Vorrichtung 10 geleitet werden, wodurch die Strahlungsquelle 14 mit ihrer Strahlung 15 nur eine geringe Schichtdicke des Mediums durchdringen muss, um eine Mindestintensität zur Aktivierung des Reinigungsfluids im erzeugten Film bereitzustellen. Grundsätzlich kann der gesamte Durchflussbehälter 11 als formgebendes Element 16 gemäß Fig. 2 vorgesehen sein. Alternativ hierzu kann ebenfalls wie nach den Figuren 3, 4 und 5 der Durchflussbehälter in einem ersten Bereich stromaufwärts von der Strahlungsquelle 14 mit einem beliebigen Durchmesser und/oder beliebiger Geometrie gebildet sein und vor oder im Bereich der Strahlungsquelle 14 mittels eines Übergangsstücks 16' zu dem formgebenden Element gemäß der Figuren 2 bis 5 umleitbar sein.

Fig. 3 zeigt das formgebende Element als Doppelrohranordnung, welche vorzugsweise koaxial zueinander angeordnet sind. Hierdurch sind ein inneres Rohr 18 und ein äußerer Rohrring 17 gebildet. In dem inneren Rohr 18 kann eine Strahlungsquelle 14 angeordnet sein. Durch den äußeren Rohrring 17 kann das zu reinigende Medium, insbesondere mit dem Reinigungsfluid, geleitet werden. Die im Inneren angeordnete Strahlungsquelle 14 kann somit das strömende Medium gleichmäßig an einer maximalen Anzahl von Punkten radial zu einer Strömungsrichtung beleuchten. Grundsätzlich können auch mehrere solcher Rohrsysteme nebeneinander parallel vorgesehen sein. Im Übrigen können die Funktionen des inneren Rohrs 18 und des äußeren Rings 17 vertauscht sein. Ebenfalls kann anstelle der Doppelrohranordnung nach Fig. 3 nur ein äußeres Rohr vorgesehen sein, durch welches das Medium strömt, und in welchem mindestens eine Strahlungsquelle 14 direkt in dem Gemisch von Medium und Reinigungsfluid vorgesehen ist. Diese kann sich insbesondere in Strömungsrichtung längs in dem Rohr erstrecken.

Fig. 4 zeigt eine Ausführungsform des erfindungsgemäßen formgebenden Elements 16, bei welchem um eine Strahlungsquelle herum mehrere das Medium leitende Rohre 20 angeordnet sind. Diese können von einem äußeren Mantelrohr 19 ummantelt sein. Vorzugsweise ist die Strahlungsquelle 14 in einem Innenbereich in etwa äquidistant zu den einzelnen vorzugsweise ringförmig angeordneten Rohren positioniert und von diesen umgeben. Hierdurch kann eine gleichmäßige Bestrahlung des Mediums in allen bereitgestellten einzelnen Rohren 20 gewährleistet werden. Grundsätzlich kann das Medium auch zusätzlich direkt in dem Mantelrohr 19 das formgebende Element 16 durchströmen, und/oder es können zusätzliche Strahlungsquellen in den Rohren 20 vorgesehen sein.

Fig. 5 zeigt eine Kombination der Ausführungsformen gemäß Fig.3 und Fig. 4. In einem Mantelrohr 21 kann eine Vielzahl von Doppelrohranordnungen 22 vorgesehen sein, in deren Zentrum jeweils eine Strahlungsquelle 14 angeordnet sein kann. Vorzugsweise sind die mehreren Doppelrohre 22 in dem Mantelrohr zumindest in einem Außenbereich gleichförmig um ein Zentrum angeordnet, wobei eine weitere Strahlungsquelle 14 ebenfalls im Zentrum des Mantelrohrs 21 vorgesehen ist. Hierdurch wird das Medium in dem jeweiligen Doppelrohr 22 sowohl durch die innere Strahlungsquelle 14 innerhalb der Doppelrohrneuordnung als auch durch die Strahlungsquelle 14 im Zentrum des Mantelrohrs 21 von mehreren Seiten bestrahlt. Grundsätzlich kann das Medium in der Doppelrohranordnung 22 so vorgesehen sein, wie hinsichtlich Fig. 3 beschrieben, nämlich dass die Strahlungsquelle und das Medium in ihrer Position vertauscht sind oder die Doppelrohranordnung als einfacheres Rohr ausgestaltet ist, wobei die in der jeweiligen Doppelrohranordnung 14 vorgesehene Strahlungsquelle direkt in dem Medium vorliegt.

Im Übrigen kann das formgebende Element auch als Leitungsbündel gebildet sein, wobei zwischen den einzelnen Leitungen Strahlungsquellen verteilt vorliegen.

Die voranstehend beschriebenen Ausführungsformen sind lediglich beispielhaft, wobei eine Vielzahl von denkbaren Alternativen möglich ist. Grundsätzlich ist allen beschriebenen und möglichen Ausführungsformen gemeinsam, dass das Medium für eine Aufteilung in Teilströme beziehungsweise eine Formgebung auf einen maximalen Durchmesser in Strömungsrichtung beschränkt werden kann, wodurch eine gleichmäßige Bestrahlung des Mediums, insbesondere des Reinigungsfluid Sendemediums und damit dessen Aktivierung begünstigt wird.

Das Reinigungsmedium kann im Gas vernebelt und so zur Entkeimung des Gases, insbesondere nach einer Strahlungsaktivierung, genutzt werden.

## Patentansprüche

1. Verfahren zur Reduzierung der Anzahl von Keimen in oder auf einem Medium, bei welchem
- ein Reinigungsfluid dem Medium zum Bilden eines Gemisches beigemengt oder auf eine Oberfläche des Mediums aufgetragen wird,
- das Gemisch oder die Oberfläche mit Reinigungsmedium mit einer elektromagnetischen Strahlung (15) mit mindestens einer definierten Wellenlänge gleichmäßig bestrahlt wird und
- das Reinigungsfluid im Zuge der Bestrahlung des Gemisches oder der Oberfläche die Keime reduziert.

2. Verfahren nach Anspruch 1,
**dadurch gekennzeichnet,**
**dass** das Gemisch als Film im Bereich einer Strahlungsquelle (14) bereitgestellt wird.

3. Verfahren nach einem der Ansprüche 1 oder 2,
**dadurch gekennzeichnet,**
**dass** der Film eine Dicke von 0,1 mm bis 30 cm, vorzugsweise von 5 mm bis 5 cm, aufweist.

4. Verfahren nach einem der Ansprüche 1 bis 3,
**dadurch gekennzeichnet,**
**dass** das Reinigungsfluid für eine Reduzierung der Keime durch die elektromagnetische Strahlung (15) aktiviert wird.

5. Verfahren nach einem der Ansprüche 1 bis 4,
**dadurch gekennzeichnet,**
**dass** die Anzahl der Keime durch einen Kontakt der Keime mit dem Reinigungsfluid reduziert wird.

6. Verfahren nach einem der Ansprüche 1 bis 5,
**dadurch gekennzeichnet,**
**dass** das Gemisch durch eine Sogwirkung oder einen erhöhten Druck einer Pumpe (13) der Strahlungsquelle zugestellt wird.

7. Verfahren nach einem der Ansprüche 1 bis 6,
**dadurch gekennzeichnet,**
**dass** eine Intensität der elektromagnetischen Strahlung (15) auf eine Mindestintensität in dem Gemisch zum Abtöten der Keime angepasst wird.

8. Verfahren nach einem der Ansprüche 1 bis 7,
**dadurch gekennzeichnet,**
**dass** das Gemisch mit einer Fördergeschwindigkeit der Quelle (14) der elektromagnetischen Strahlung (15) zugestellt wird, welche an die Intensität der elektromagnetischen Strahlung (15) angepasst wird.

9. Vorrichtung zur Reduzierung von Keimen in einem fluiden Medium, ,
mit
- mindestens einem Durchflussbehälter (11), durch den das Medium mit beigemischtem Reinigungsfluid transportierbar ist, und
- mindestens einer Strahlungsquelle (14), die ausgebildet ist, das transportierte Gemisch mit einer Strahlung (15) definierter Wellenlänge gleichmäßig zu bestrahlen.

10. Vorrichtung nach Anspruch 9,
**dadurch gekennzeichnet,**
**dass** der Durchflussbehälter (11) ausgebildet ist, das keimbelastete Medium im Bereich der Strahlungsquelle (14) zu fördern, wobei ein formgebendes Element (16) vorgesehen ist, welches das keimbelastete Medium im Bereich der Strahlungsquelle als Film bereitstellt.

11. Vorrichtung nach einem der Ansprüche 9 oder 10,
**dadurch gekennzeichnet,**
**dass** das formgebende Element (16) als Doppelschlitz ausgebildet ist, durch welchen das Medium förderbar ist.

12. Vorrichtung nach einem der Ansprüche 9 bis 11,
**dadurch gekennzeichnet,**
**dass** das formgebende Element (16) als koaxiales Doppelrohr ausgebildet ist, und/oder die Strahlungsquelle (14) im inneren Rohr (18) vorgesehen ist und das Öl durch das äußere Rohr (17) förderbar ist.

13. Vorrichtung nach einem der Ansprüche 9 bis 12,
**dadurch gekennzeichnet,**
**dass** das formgebende Element (16) als eine Anordnung von mehreren Rohren (19) ausgebildet ist, durch welche das Medium förderbar ist.

14. Vorrichtung nach einem der Ansprüche 9 bis 13,
**dadurch gekennzeichnet,**
**dass** die mehreren Rohre (19), das innere Rohr (18) des Doppelrohrs beziehungsweise der Doppelschlitz, zumindest im Bereich der Strahlungsquelle, strahlungsdurchlässig für die Strahlung (15) der mindestens einen Strahlungsquelle (14) sind.

15. Vorrichtung nach einem der Ansprüche 9 bis 14,
**dadurch gekennzeichnet,**
**dass** mindestens eine Strahlungsquelle (14) zumindest in einem Bereich des Druchflussbehälters (11) vorgesehen ist.
